# EUROPÄISCHE PATENTSCHRIFT

(11) **EP 0 670 830 B1**
(45) Veröffentlichungstag und Bekanntmachung des Hinweises auf die Patenterteilung: **27.06.2001**
(21) Anmeldenummer: 94901844.4
(22) Anmeldetag: 22.11.1993
(51) Int. Cl.: C07D 233/14, C07C 233/36, C11D 1/88

(54) **VERFAHREN ZUR HERSTELLUNG DÜNNFLÜSSIGER, LAGERSTABILER AMPHOTENSIDE**
PROCESS FOR PREPARING FLUID, STORAGE-STABLE AMPHOLYTIC SURFACE-ACTIVE AGENTS
PROCEDE DE PREPARATION D'AGENTS DE SURFACE AMPHOLYTES FLUIDES ET STABLES AU STOCKAGE

(30) Priorität: 30.11.1992 DE 4240154
(43) Veröffentlichungstag der Anmeldung: 13.09.1995
(73) Patentinhaber: Cognis Deutschland GmbH, 40589 Düsseldorf (DE)
(72) Erfinder: UPHUES, Günter, D-40789 Monheim (DE); PLOOG, Uwe, D-42781 Haan (DE); SCHICK, Renate, D-42781 Haan (DE); SCHWARK, Hans-Jürgen, D-40789 Monheim (DE); WITT, Sandra, D-40764 Langenfeld (DE)
(86) Internationale Anmeldenummer: EP9303273
(87) Internationale Veröffentlichungsnummer: WO9412477

(56) Entgegenhaltungen:
- DE-A- 2 063 424
- DE-A- 3 641 871
- DE-A- 4 038 983
- US-A- 4 269 730

## Beschreibung

### Gebiet der Erfindung

Die Erfindung betrifft ein Verfahren zur Herstellung von dünnflüssigen, lagerstabilen Amphotensiden, bei dem man Imiazoline innerhalb enger pH-Wertbereiche quaterniert bzw. carboxymethyliert, hydrolysiert und endlagert.

### Stand der Technik

Amphotere Tenside, insbesondere vom Typ der Imidazoliniumbetaine,weisen gute Schaum- und Reinigungseigenschaften auf und gewinnen als Co-Tenside in Handgeschirrspülmitteln sowie kosmetischen Reinigungs- und Pflegemitteln zunehmend an Bedeutung [**Seifen-Fette-Öle-Wachse, 108, 373 (1982)**]. Üblicherweise werden derartige Tenside durch Alkylierung von Imidazolinen mit Hilfe von Natriumchloracetat und nachfolgende bzw. gleichzeitige Hydrolyse des Imidazolinrings hergestellt. Ein besonderes Problem bei der Herstellung besteht darin, amphotere Imidazoliniumtenside zu erhalten, die auch bei längerer Lagerung niedrigviskos und somit leicht pump- und dosierbar sind.

In der Vergangenheit hat es nicht an Versuchen gemangelt, amphotere Tenside vom Imidazolinbetain-Typ herzustellen. In diesem Zusammenhang lassen sich zwei Routen unterscheiden:
1. Einstufiges Verfahren: Die Umsetzung mit Natriumchloracetat findet erst statt, nachdem die Hydrolyse des Imidazolinrings stattgefunden hat [vgl. **GB-A 850.514, GB 930.296, US 2.773.068, EP-B 0 040 346** (Henkel), **DE 36 39 752** (Kao)]. Gemäß der Lehre der genannten Schriften werden jedoch stets hochviskose Produkte mit einem angestrebten Feststoffgehalt von ca. 50 Gew.-% erhalten.
2. Zweistufiges Verfahren: Im ersten Schritt wird das Imidazolin zum Betain umgesetzt, welches anschließend in Gegenwart von Basen hydrolysiert, d.h. ringgeöffnet wird, um dann mit weiterem Natriumchloracetat abzureagieren [vgl. **DE 20 63 424** (Rewo), **US 4.269.730** (Stepan), **EP-B-0 001 006** (Albright & Wilson), **DE 40 38 983** (Henkel)]. Auf diese Weise hergestellte Produkte sind zwar unmittelbar nach der Herstellung auch hochkonzentriert dünnflüssig, bei längerer Lagerung steigt die Viskosität jedoch rasch an und führt schließlich zu nicht mehr fließfähigen, gallertartigen Substanzen.

Aus einer umfangreichen Arbeit von S.Takano und K.Tsuji in **J.Am.Oil.Chem.Soc. 60, 1807 (1983)** ist bekannt, daß die hochviskosen Produkte einen hohen Gehalt an offenkettigen, monocarboxylierten Verbindungen der Formel **(IIa)** enthalten:

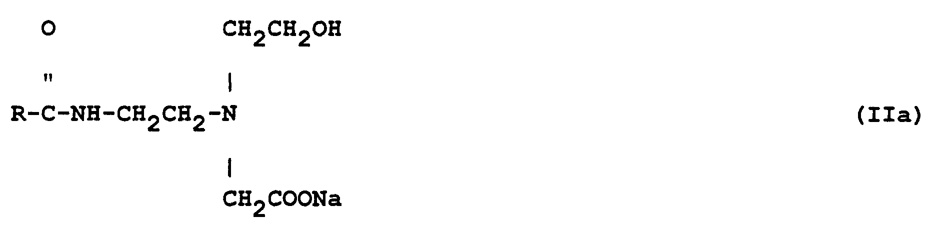

In niedrigviskosen Produkten wird hingegen als Hauptprodukt ein dicarboxyliertes Produkt der Formel **(IIb)** gefunden:

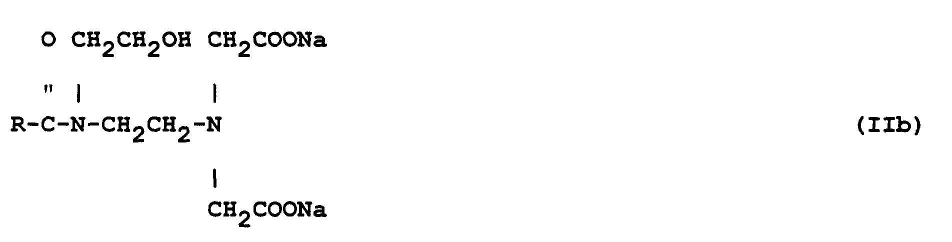

Die Aufgabe der Erfindung bestand somit darin, ein verbessertes Verfahren zur Herstellung von dünnflüssigen, lagerstabilen Amphotensiden zu entwickeln. Insbesondere sollten Amphotenside mit einem möglichst hohen Gehalt von Verbindungen des Typs **(IIb)** zugänglich gemacht werden.

### Beschreibung der Erfindung

Gegenstand der Erfindung ist ein Verfahren zur Herstellung dünnflüssiger, lagerstabiler Amphotenside, bei dem man
a) 1-Hydroxyethyl-2-alkyl-2-imidazoline der Formel (**I**), 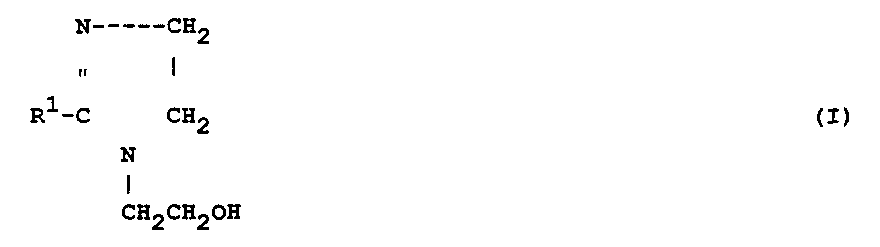 in der R¹ für einen Alkylrest mit 5 bis 21 Kohlenstoffatomen steht, bei einem pH-Wert im Bereich von 7,5 bis 9 und genauer Kontrolle des pH-Wertes mit halogenierten Carbonsäuresalzen quaterniert bzw. carboxymethyliert und gleichzeitig mit wäßrigen Basen hydrolysiert sowie
b) die Reaktionsendprodukte auf einen pH = 5 bis 7 einstellt.

Überraschenderweise wurde gefunden, daß die genaue Kontrolle des pH-Wertes sowohl bei der Herstellung der Imidazoliniumbetaine, als auch bei ihrer Lagerung, Produkte liefert, die auch hochkonzentriert dünnflüssig sind und selbst bei wochenlanger Lagerung eine konstant niedrige Viskosität aufweisen.

### Ausgangsstoffe

**1-Hydroxyethyl-2-alkyl-2-imidazoline** stellen bekannte Stoffe dar, die man beispielsweise durch Kondensation von Fettsäuren mit Aminoethylethanolamin erhält. Typische Beispiele für Imidazoline, die im Sinne des erfindungsgemäßen Verfahrens als Ausgangsstoffe in Betracht kommen, sind die Kondensationsprodukte von Aminoethylethanolamin mit Capronsäure, Caprylsäuren, Caprinsäure, Laurinsäure, Myristinsäure, Palmitinsäure, Stearinsäure, Isostearinsäure, Arachinsäure und Behensäure sowie deren technische Mischungen, wie sie beispielsweise bei der Druckspaltung von nativen Fetten und Ölen anfallen. Vorzugsweise werden Imidazoline der Formel **(I)** auf Basis von technischen Kokos- oder Talgfettsäuren eingesetzt, in der R¹ für einen Alkylrest mit 11 bis 17 Kohlenstoffatomen steht.

Unter **halogenierten Carbonsäuresalzen** sind die Natrium- und/ oder Kaliumsalze der Halogenessigsäure, Halogenpropionsäure und/oder Halogenbuttersäure zu verstehen. Vorzugsweise wird Natriumchloracetat eingesetzt.

### Quaternierung, Carboxymethylierung und Hydrolyse

Üblicherweise können die Imidazoline und die halogenierten Carbonsäuresalze in molaren Mengen von 1 : 1,5 bis 1 : 3, vorzugsweise 1 : 1,8 bis 1 : 2,5 eingesetzt werden. Als optimal hat es sich erwiesen, die Quaternierung bzw. Carboxymethylierung sowie die Hydrolyse gleichzeitig bei Temperaturen im Bereich von 70 bis 85°C, vorzugsweise 78 bis 83°C durchzuführen. Als wäßrige Basen kommen Natron- und/oder Kalilauge in Betracht; vorzugsweise werden 5 bis 55 und insbesondere 30 bis 50 gew.-%ige Lösungen von Natriumhydroxid eingesetzt. Die Menge an Base richtet sich nach dem Gehalt an halogeniertem Carbonsäuresalz. Vorzugsweise wird man Base und Salz in einem molaren Verhältnis von 0,9 : 1 bis 1 : 1,2, vorzugsweise 1 : 1 bis 1 : 1,1 einsetzen. Aufgabe der Base ist es, mit dem Halogenanteil des Carbonsäuresalzes ein anorganisches Salz , z.B. Natriumchlorid, zu bilden. Wenn es in der Praxis dennoch vorteilhaft sein sollte, über das äquimolare Verhältnis hinauszugehen, so dann, wenn zur Aufrechterhaltung des als kritisch erkannten pH-Bereiches eine höhere Basenkonzentration erforderlich ist. Zum Puffern der Mischungen hat es sich ferner als vorteilhaft erwiesen, den Lösungen beispielsweise geringe Mengen Citronensäure zuzusetzen.

Zur Verdeutlichung der der Erfindung zugrundeliegenden Erkenntnisse, soll das Verfahren an dieser Stelle exemplarisch beschrieben werden:

Eine Lösung von wäßrigem Natriumchloracetat und Citronensäure wird vorgelegt. Beginnend bei 40°C wird das Imidazolin innerhalb von 30 min zugegeben, wobei die Temperatur auf ca. 50°C und der in der Mischung gemessene pH-Wert auf 11,65 ansteigt. Anschließend wird die Temperatur rasch auf 70°C erhöht, wobei der pH-Wert absinkt. Im Sinne des erfindungsgemäßen Verfahrens wird nun der pH-Wert durch Zugabe wäßriger Natronlauge bei 8,5 konstant gehalten. Die HPLC-Analyse einer zu diesem Zeitpunkt entnommenen Probe zeigt, daß Mono- und und überwie- gend Dicarboxylat nebeneinander vorliegen. Nach einer Reaktionszeit von ca 7 h und einem Verbrauch von 90 Gew.-% der insgesamt zur Bildung des anorganischen Salzes erforderlichen Menge Natronlauge, zeigt das Chromatogramm nur noch geringe Anteile nicht abreagierten Imidazolins. Die Restmenge an Base wird in einer Portion zugegeben. Die Analyse des Reaktionsendproduktes, das durch Zugabe von Säure auf pH = 8,5 eingestellt wird, weist ein Verhältnis Di/Monocarboxylat von größer 6 auf; die Viskosität liegt unter 100 mPas. Zur Lagerung wird der pH-Wert auf 6 abgesenkt.

Wird die Reaktion bei pH-Werten größer 9 durchgeführt, kann man mit Hilfe der HPLC feststellen, daß der größte Teil des Imidazolins hydrolysiert, ehe eine Quaternierung stattfinden kann. Die parallel verlaufende Carboxylmethylierung liefert ein hochviskoses Produkt, das überwiegend Verbindungen vom Typ **(IIa)** enthält.

Wird die Reaktion bei pH-Werten kleiner 7,5 durchgeführt, beobachtet man die Einstellung eines Gleichgewichtes aus betainisiertem und freiem Imidazolin. Bei Zugabe von Natronlauge findet eine rasche Hydrolyse beider Species statt, wobei wieder ein hoher Anteil des Verbindungstyps **(IIa)** resultiert (Verhältnis Di/Monocarboxylat < 3).

Wird die Reaktion hingegen im Sinne des erfindungsgemäßen Verfahrens durchgeführt - pH-Bereich 7,5 bis 9 - stellt sich zwar ebenfalls ein Gleichgewicht zwischen betainisiertem und freiem Imidazolin ein, unter den Reaktionsbedingungen wird jedoch fast ausschließlich das betainisierte Imidazolin ringgeöffnet. Entsprechend der Gleichgewichtslage wird aus dem freien Imidazolin Betain nachgebildet, welches seinerseits wieder hydrolysieren kann. In Summe wird also vorwiegend der niedrigviskose Verbindungstyp **(IIb)** gebildet.

### Lagerstabilität

Die auf Basis Imidazolin erhaltenen Amphotensid-Konzentrate des Stands der Technik zeigen bei Lagerung praktisch ausnahmslos eine stetig fortschreitende Viskositätserhöhung, deren Geschwindigkeit von den Lagerbedingungen, insbesondere jedoch vom Verhältnis der Mono- und Dicarboxylate **(IIa)** und **(IIb)** abhängt. Typischerweise nimmt das Di/Monocarboxylat-Verhältnis nach einer Lagerzeit von 4 Wochen bei 60°C bei Produkten des Stands der Technik von 3,2 auf 1,4 ab; hieraus resultiert ein Viskositätsanstieg auf mehr als das Vierfache des Ausgangswertes. Der Hydrolyseempfindlichkeit von **(IIb)** kann im Sinne der Erfindung begegnet werden, indem man das Produkt zur Lagerung auf einen pH-Wert von 5 bis 7 einstellt. HPLC-Untersuchungen haben ergeben, daß unter diesen Bedingungen das Di/Monocarboxylat-Verhältnis sowie der Gehalt an freier Fettsäure auch bei längerer Lagerung konstant bleiben.

### Gewerbliche Anwendbarkeit

Die nach dem erfindungsgemäßen Verfahren erhältlichen Amphotenside sind dünnflüssig und auch über längere Zeit lagerstabil. Sie eignen sich zur Herstellung oberflächenaktiver Mittel, insbesondere Spül- und Reinigungsmittel sowie Produkten zur Haar- und Körperpflege, in denen sie in Mengen von 0,1 bis 25, vorzugsweise 0,5 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

In einer 400-ml-Vierhals-Rührapparatur mit Rückflußkühler, Thermometer, pH-Elektrode und Tropftrichter wurden 73,9 g (633 mmol) Natriumchloracetat, gelöst in 152,8 g Wasser, 0,58 g Citronensäure-Monohydrat und 100 g (352 mmol) eines Imidazolins zur Reaktion gebracht, welches wie in der DE-A 36 41 871 angegeben aus einer hydrierten C12/18-Kokosfettsäure und Aminoethylethanolamin erhalten wurde. Zur Steuerung des pH-Wertes wurden insgesamt 152,8 g (633,8 mmol) Natriumhydroxid in Form einer 50 gew.- %igen wäßrigen Lösung verbraucht. Das Endprodukt - eine klare Flüssigkeit - wurde mit konzentrierter Salzsäure auf einen pH-Wert von 6,5 eingestellt.

| Kenndaten des Produktes: | |
|---|---|
| Viskosität | 40 mPas |
| Di/Monocarboxylat-Verhältnis | 8,9 |
| Wassergehalt (nach Fischer) | 49,8 Gew.-% |

### Beispiel 2:

In einer 400-ml-Vierhals-Rührapparatur mit Rückflußkühler, Thermometer, pH-Elektrode und Tropftrichter wurden 77,7 g (666 mmol) Natriumchloracetat in 170,2 g Wasser gelöst. Nach Zugabe von 0,6 g Citronensäure-Monohydrat wurden bei 40°C innerhalb von 25 min 100 g (371 mmol) 1-Hydroxyethyl-2-undecyl-2-imidazolin gleichmäßig zugesetzt, wobei die Temperatur auf 50°C und der pH-Wert (gemessen in der Reaktionsmischung) auf 11,7 anstieg. Anschließend wurde die Reaktionsmischung rasch auf 70°C erhitzt, wobei ein Abfall des pH-Wertes beobachtet werden konnte; durch Zugabe von 38,9 g (486 mmol) Natriumhydroxid in Form einer 50gew.-%igen wäßrigen Lösung wur- de der pH-Wert bei 8,5 konstant gehalten und die Mischung über 240 min gerührt. Anschließend wurde der pH-Wert auf 9,0 erhöht und weitere 180 min durch Zugabe von Natronlauge konstant gehalten; der gesamte Verbrauch an NaOH betrug bis zu diesem Zeitpunkt 47,5 g (593,7 mmol). Nach weiteren 120 min - der pH-Wert war inzwischen auf 8,3 abgesunken - wurden nochmals 5 g (62,5 mmol) Natronlauge zugesetzt und 60 min gerührt. Nach Abschluß der Reaktion wurden 1500 g des erhaltenen Produktes in 6 Portionen geteilt und mit Salzsäure bzw. Natronlauge auf pH-Werte im Bereich von 3 bis 12 eingestellt.

Gleiche Anteile dieser Produkte wurden 2 Wochen lang bei Temraturen im Bereich von 5 bis 60°C gelagert. Danach wurden das Di/Monocarboxylat-Verhältnis, der Fettsäuregehalt und die Viskosität analysiert. Die Ergebnisse sind in Tab.1 zusammengefaßt.

### Vergleichsbeispiel V1:

Wie in der Patentschrift **DE-B 40 38 983,** Beispiele 1 und 9 beschrieben, wurden 237,8 g (2,04 mol) Natriumchloracetat, gelöst in 450 g Wasser nach Zusatz von 8,4 g Citronensäure mit 268 g (1 mol) 1-Hydroxyethyl-2-undecyl-2-imidazolin umgesetzt. Nach Beendigung der Imidazolinzugabe wurde die Mischung über 30 min bei 80°C gerührt. Anschließend wurde über einen Zeitraum von 120 min gleichmäßig 155,7 g (1,95 mol) Natriumhydroxid in Form einer 50 gew.-%igen wäßrigen Lösung zugesetzt. Nach weiteren 180 min Reaktionszeit wurde durch Zugabe 50 gew.-%iger Citronensäure ein pH-Wert von 8,25 eingestellt und das Produkt abgekühlt. Durch Zugabe von Wasser wurde ein Feststoffgehalt von 50,0 Gew.-% eingestellt.

Untersuchungen zur Lagerstabilität sind in Tab.2 zusammengefaßt.

**Tab.1:**

| Lagerverhalten erfindungsgemäßes Beispiel 2 | | | | |
|---|---|---|---|---|
| pH-Wert | T °C | DMV | Fettsäure Gew.-% | Viskos. mPas |
| 3,05 | 5 | 7,2 | 0,24 | 5100 |
| | 25 | 6,7 | 0,35 | 4500 |
| | 60 | 4,5 | 2,70 | 16300 |
| 5,07 | 5 | 8,3 | 0,29 | 280 |
| | 25 | 8,2 | 0,24 | 270 |
| | 60 | 6,6 | 0,40 | 1210 |
| 7,04 | 5 | 8,3 | 0,22 | 280 |
| | 25 | 8,2 | 0,25 | 250 |
| | 60 | 5,3 | 0,35 | 2150 |
| 8,59 | 5 | 8,2 | 0,24 | 155 |
| | 25 | 8,1 | 0,27 | 120 |
| | 60 | 3,3 | 1,00 | 12500 |
| 10,46 | 5 | 8,4 | 0,32 | 130 |
| | 25 | 8,4 | 0,34 | 120 |
| | 60 | 4,2 | 2,50 | 21000 |
| 12,0 | 5 | 4,1 | 2,60 | 35000 |
| | 25 | 3,8 | 3,50 | n.m. |
| | 60 | 0,7 | 8,4 | n.m. |
| Legende: T = Temperatur DMV = Verhältnis Di/Monocarboxylat Viskos. = Viskosität Kegel/Platte-System bei 25°C mit Viskosimeter Fa.Carri-Med Ltd. n.m. = nicht meßbar | | | | |

**Tab.2:**

| Lagerverhalten Vergleichsbeispiel | | | | | |
|---|---|---|---|---|---|
| pH-Wert | T °C | Lt w | DMV | Fettsäure Gew.-% | Viskos. mPa.s |
| 8,25 | - | - | 3,2 | 1,1 | 380 |
| 8,10 | 60 | 1 | 2,6 | 2,1 | 16000 |
| 7,90 | 60 | 4 | 1,4 | 4,3 | 40000 |
| Legende: Lt = Lagerzeit w = Wochen | | | | | |

## Patentansprüche

1. Verfahren zur Herstellung dünnflüssiger, lagerstabiler Amphotenside, bei dem man
a) 1-Hydroxyethyl-2-alkyl-2-imidazoline der Formel **(I),** 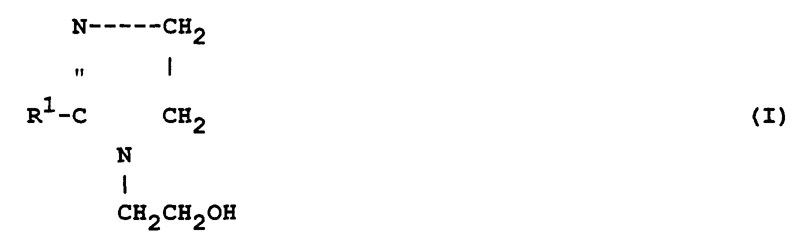 in der R¹ für einen Alkylrest mit 5 bis 21 Kohlenstoffatomen steht, bei einem pH-Wert im Bereich von 7,5 bis 9 und genauer Kontrolle des pH-Wertes mit halogenierten Carbonsäuresalzen quaterniert bzw. carboxymethyliert und gleichzeitig mit wäßrigen Basen hydrolysiert sowie
b) die Reaktionsendprodukte auf einen pH = 5 bis 7 einstellt.

2. Verfahren nach Anspruch 1, **dadurch gekennzeichnet,** daß man Imidazoline der Formel **(I)** einsetzt, in der R¹ für einen Alkylrest mit 11 bis 17 Kohlenstoffatomen steht.

3. Verfahren nach Anspruch 1, **dadurch gekennzeichnet,** daß man als halogeniertes Carbonsäuresalz Natriumchloracetat einsetzt.

4. Verfahren nach Anspruch 1, **dadurch gekennzeichnet,** daß man die Imidazoline und die halogenierten Carbonsäuresalze in molaren Mengen von 1 : 1,5 bis 1 : 3 einsetzt.

5. Verfahren nach Anspruch 1, **dadurch gekennzeichnet,** daß man die Quaternierung bzw. Carboxymethylierung sowie die Hydrolyse gleichzeitig bei Temperaturen im Bereich von 70 bis 85°C durchführt.

6. Verfahren nach Anspruch 1, **dadurch gekennzeichnet,** daß daß man als wäßrige Basen 5 bis 55 gew.-%ige Lösungen von Natriumhydroxid einsetzt.

7. Verfahren nach Anspruch 1, **dadurch gekennzeichnet,** daß man die halogenierten Carbonsäuresalze und die Basen im molaren Verhältnis von 0,9 : 1 bis 1 : 1,2 einsetzt.

## Claims

1. A process for the production of low-viscosity, storable amphoteric surfactants in which
a) 1-hydroxyethyl-2-alkyl-2-imidazolines corresponding to formula (I): 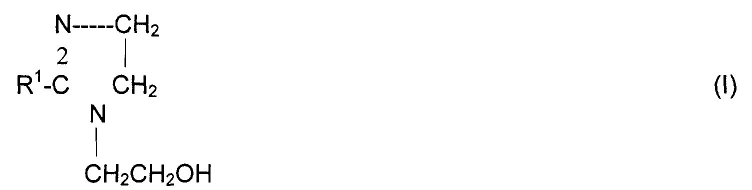 in which R¹ is an alkyl group containing 5 to 21 carbon atoms, are quaternized or carboxymethylated with halogenated carboxylic acid salts and, at the same time, hydrolyzed with aqueous bases at a strictly controlled pH value in the range from 7.5 to 9 and
b) the end reaction products are adjusted to a pH value of 5 to 7.

2. A process as claimed in claim 1, characterized in that imidazolines corresponding to formula (I), in which R¹ is an alkyl group containing 11 to 17 carbon atoms, are used.

3. A process as claimed in claim 1, characterized in that sodium chloroacetate is used as the halogenated carboxylic acid salt.

4. A process as claimed in claim 1, characterized in that the imidazolines and the halogenated carboxylic acid salts are used in molar quantities of 1:1.5 to 1:3.

5. A process as claimed in claim 1, characterized in that the quaternization or carboxymethylation and the hydrolysis are carried out simultaneously at temperatures of 70 to 85EC.

6. A process as claimed in claim 1, characterized in that 5 to 55% by weight solutions of sodium hydroxide are used as the aqueous bases.

7. A process as claimed in claim 1, characterized in that the halogenated carboxylic acid salts and the bases are used in a molar ratio of 0.9:1 to 1:1.2.

## Revendications

1. Procédé de préparation d'agents de surface amphotères fluides, stables au stockage, dans lequel :
a) des 1-hydroxyéthyl-2-alkyl-2-imidazolines de formule I, 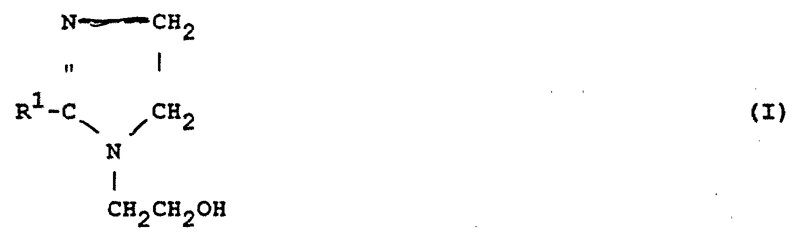 dans laquelle R¹ représente un radical alkyle ayant de 5 à 21 atomes de carbone,
sont quaternisés ou carboxyméthylés à une valeur de pH dans la zone de 7,5 à 9 et sous contrôle précis de la valeur du pH, avec des sels d'acide carboxylique halogénés, et en même temps sont hydrolysés avec des bases aqueuses, ainsi que
b) on ajuste à un pH = 5 à 7 les produits finaux de réaction.

2. Procédé selon la revendication 1,
caractérisé en ce qu'
on met en oeuvre des imidazolines de formule (I) dans laquelle R¹ représente un radical alkyle ayant de 11 à 17 atomes de carbone.

3. Procédé selon la revendication 1,
caractérisé en ce qu'
on met en oeuvre comme sel d'acide carboxylique halogéné, du chloracétate de sodium.

4. Procédé selon la revendication 1,
caractérisé en ce qu'
on met en oeuvre l'imidazoline et les sels d'acide carboxylique halogéné en quantités molaires allant de 1:1,5 à 1:3.

5. Procédé selon la revendication 1,
caractérisé en ce qu'
on effectue la quaternisation ou la carboxyméthylation ainsi que l'hydrolyse simultanément à des températures dans la plage de 70 à 85°C.

6. Procédé selon la revendication 1,
caractérisé en ce qu'
on met en oeuvre en tant que bases aqueuses des solutions de 5 à 55 % en poids d'hydroxyde de sodium.

7. Procédé selon la revendication 1,
caractérisé en ce qu'
on met en oeuvre les sels d'acide carboxylique halogénés et les bases dans un rapport molaire allant de 0,9:1 à 1:1,2.
